# EUROPÄISCHE PATENTSCHRIFT

(11) **EP 0 781 999 B1**
(45) Veröffentlichungstag und Bekanntmachung des Hinweises auf die Patenterteilung: **04.10.2001**
(21) Anmeldenummer: 96119064.2
(22) Anmeldetag: 28.11.1996
(51) Int. Cl.: G01N 33/543

(54) **Massensensitive Biosensoren**
Mass-sensitive biosensors
Biosensors massiques-sensibles

(30) Priorität: 27.12.1995 DE 19548376
(43) Veröffentlichungstag der Anmeldung: 02.07.1997
(73) Patentinhaber: Dade Behring Marburg GmbH, 35001 Marburg (DE)
(72) Erfinder: Wiegand, Andreas, 34641 Schwalmstadt (DE); Madry, Norbert, Dr., 35041 Marburg (DE); Schelp, Carsten, Dr., 35041 Marburg (DE); Meller, Paul, Dr., 35041 Marburg (DE); Möhlen, Michael, 35041 Marburg (DE)

(56) Entgegenhaltungen:
- DE-A- 4 436 910
- US-A- 4 236 893
- US-A- 5 314 830
- JOURNAL OF THE AMERICAN CHEMICAL SOCIETY, Bd. 110, Nr. 26, 1988, WASHINGTON DC USA, Seiten 8623-8623, XP000647968 R. C. EBERSOLE ET AL.: "Amplified mass immunosorbent assay with a quartz crystal microbalance."

## Beschreibung

Die vorliegende Erfindung betrifft einen piezoelektrischen Sensor zur Verwendung in diagnostischen und analytischen Verfahren insbesondere zum immunchemischen Nachweis diagnostisch relevanter spezifischer Bindungspartner.

Piezoelektrische Sensoren als solche sind z. B. aus Dickert et al. (Chemie in unserer Zeit 28 (3), S. 147 - 152 (1994)) bekannt. Solche Biosensoren sind z. B. auch in den US Patentschriften 4,236,893 und 4,735,906 beschrieben.

Solche Biosensoren, die zum Nachweis von diagnostisch relevanten Molekülen (Analyten) verwendet werden können, bestehen in der Regel aus einem Transducer mit einer mit einem für das nachzuweisende Molekül spezifischen Bindungspartner beschichteten Oberfläche, die dadurch in der Lage ist, selektiv diese Moleküle zu binden. Als Transducer werden meistens piezoelektrische Quarzkristalle verwendet. Wird der Quarzkristall in einen entsprechenden elektronischen Schaltkreis eingebaut, so schwingt er in einer bestimmten Rescnanzfrequenz. Lagern sich nun die nachzuweisenden Moleküle an, so verschiebt sich die Resonanzfrequenz. Solche elektronischen Schaltkreise sind an sich dem Fachmann bekannt, z. B. aus der DE-A 39 20 052.

Auch wenn diese bekannten Biosensoren die an sie gestellten Aufgaben bereits zum Teil erfüllen, so zeigt sich doch, daß bei den verwendeten Verfahren. bei denen z. B. Antikörper über Silanderivate an die Quarzoberfläche gebunden werden, daß zum einen das Verfahren zur Bindung der Antikörper an die Quarzoberfläche sehr aufwendig und schlecht reproduzierbar ist und zum anderen das Verfahren zur Wiederaufbereitung der Sensoroberfläche durch behandeln mit einer Reagenzlösung, die die Bindung zwischen nachzuweisendem Antigen und Antikörper aufbrechen und das gebundene Antigen ablösen soll, seinerseits schlecht reproduzierbar ist und zu einem unkontrollierten Abbau der Antikörper auf der Sensoroberfläche führt.

In einer Veröffentlichung von DAVIS et al. (Anal.Chem. (1989), 61 (11), S. 1227 - 1230) wird beschrieben, daß man den Transducer mit Gold beschichten kann und an diese Schicht Protein A binden. Das Protein A kann seinerseits reversibel Antikörper zum Nachweis diagnostisch relevanter Moleküle binden. Wie die Autoren selber ausführen, wird das Signal durch die Beschichtung mit Protein A stark verschoben und die Messung insgesamt erschwert.

In einer Veröffentlichung von Ebersole und Ward (J. Am. Chem. Soc. (1988), 110: 8623-8628) ist ein Verfahren zum Nachweis der APS Reductase beschrieben, bei dem ein Anti-APS Reductase-Antikörper direkt an die Goldelektrode eines piezoelektrischen Sensors gebunden werden kann. Ferner ist hierin auch die Wiederverwendbarkeit von PV-Fc-Film beschichteten Sensoren in einem Nachweisverfahren zur Bestimmung von hCG erläutert. Hierbei werden die spezifischen Bindungspartner nicht direkt an einen edelmetallbeschichteten. Sensor sondern an eine separate Nylonmembran gebunden. In US 5,314,830 wird ein wiederverwendbarer piezoelektrische Sensor beschrieben, an den ein Antikörper gebunden wird, der so modifiziert wurde, daß der Antikörper selbst bei Zugabe von chaotropen Reagenzien nicht von der Sensoroberfläche abgelöst wird. Ein Nachteil dieses Sensors ist, daß die Testqualität nach mehrmaliger Nutzung aufgrund zunehmender Denaturierung des ersten Bindungspartners abnimmt.

Der vorliegenden Erfindung liegt somit die Aufgabe zugrunde, einen Biosensor zur Verwendung in analytischen oder diagnostischen Verfahren vorzuschlagen, der einfach mit einem spezifischen Bindungspartner zu belegen ist und zugleich nach Ablauf der spezifischen Reaktion einfach und zuverlässig zu regenerieren ist.

Diese Aufgabe wird gemäß der vorliegenden Erfindung dadurch gelöst, daß der piezoelektrische Sensor mit einem Edelmetall, bevorzugterweise Gold beschichtet wird und an diese Beschichtung ein erster spezifischer Bindungspartner direkt gebunden werden kann. Von dem so beschichteten Biosensor kann z. B. durch die aus der DE 44 36 910 bekannten Reagenzien dieser spezifische Bindungspartner abgelöst werden.

Für die vorliegende Erfindung ist es nicht wesentlich, in welcher Ausführungsform der Transducer vorliegt oder auf welche Art und Weise die durch die Anlagerung von Analyten bewirkte Veränderung in ein Meßsignal umgewandelt wird. Die vorliegende Erfindung kann sowohl in manuellen Verfahren, bei denen z. B. der Sensor an die Probe eingetaucht wird, als auch in Analysenautomaten eingesetzt werden.

Grundsätzlich kann das erfindungsgemäße Verfahren wie folgt durchgeführt werden: Ein handelsüblicher goldbeschichteter Piezokristall, - der in einen geeigneten elektronischen Schaltkreis integriert eingebaut ist - (z. B. von der Fa. Sensotec) wird mit einem für den zu bestimmenden Analyten spezifischen Bindungspartner beschichtet. Ein solcher spezifischer Bindungspartner kann z. B. ein mono- oder polyklonaler Antikörper oder Antikörperfragment, ein Lectin oder ein Antigen sein. Die Beschichtungsdauer kann zwischen 5 min. und mehreren Stunden, bevorzugterweise zwischen 10 und 120 min. liegen. Nach der Beschichtung wird die Sensoroberfläche mit Waschpuffer gespült. Der Waschpuffer hat vorzugsweise ein Detergenz. Gegebenenfalls kann nach der spezifischen Beschichtung noch eine unspezifische Beschichtung mit z. B. BSA oder inaktivierter POD erfolgen, um unspezifische Bindungen zu verhindert. Solche Verfahren sind dem Fachmann an sich bekannt. Auch nach einer solchen unspezifischen Nachbeschichtung kann ein Waschschritt erfolgen.

Der beschichtete Sensor wird mit der Probe inkubiert, wobei dies z. B. durch Eintauchen in die Probe oder durch Auftragen einer Probe auf die Sensoroberfläche erfolgen kann. Vorteilhaft ist auch eine Ausführungsform, bei der die Sensoroberfläche so geformt ist, daß im Durchfluß gemessen werden kann. Es ist für den Fachmann leicht ersichtlich, welche Form der jeweilige Sensor je nach seinem Einsatz haben muß.

Durch die Reaktion des Analyten mit dem spezifischen Bindungspartner verschiebt sich die durch den elektronischen Schaltkreis bestimmte Meßgröße, z. B. die Resonanzfrequenz. Aus der Veränderung der Meßgröße kann die Menge des gebundenen Analyten, z. B. durch Vergleich mit einer Referenzkurve abgeleitet werden. Die Anordnung ist auch geeignet, direkt in Masseeinheiten geeicht zu werden. Um die Veränderung der Meßgröße zu bestimmen, kann man z. B. eine Referenzelektrode, die nicht dem spezifischen Bindungspartner beschichtet ist, verwenden. Vorteilhafterweise erfolgt nach Abschluß der Inkubation mit der Probe noch ein weiterer Waschschritt. Die Inkubation mit der Probe kann vorteilhafterweise zwischen 1 und 100 min., besonders vorteilhafterweise zwischen 5 und 60 min., ganz besonders vorteilhafterweise zwischen 10 und 30 min. erfolgen.

Die Regenerierung kann wie in der DE 44 36 910 beschrieben erfolgen. Dabei werden bei der Verwendung von Edelmetallen, vorzugsweise Gold als Festphase, bestimmte Reduktions- oder Oxidationsmittel, wie z. B. Natriumborhydrid, Tetrabutylammoniumhydroxid mit oder ohne Zusatz von Detergenzien zur Regenerierung eingesetzt.

Das folgende Ausführungsbeispiel dient der Verdeutlichung, nicht der Einschränkung der Erfindung.

### Beispiel

### Quantitative human-IgE-Bestimmung

### Träger:

Goldbeschichteter Piezokristall der Firma Sensotec, eingefaßt in einem Teflonring (Außendurchmesser 36 mm). Durchmesser der Goldoberfläche: 9 mm Goldoberfläche: 14 mm².

### Beschichtung:

Auf den Sensor werden 50 µl eines polyklonalen Antikörpers (Kaninchen) gegen human IgE gegeben. Die Konzentration des AK beträgt 5 µg/ml. Die Lösung enthält außerdem 75 mM Na-Phosphat, 75 mM NaCI, 100 g/l Na₂SO₄. Der pH-Wert ist 6,0. Die AK-Lösung wird 1 Stunde bei 37 °C , bzw. über Nacht bei Raumtemperatur belassen.

### Waschschritt:

Der Überstand wird entnommen und der Sensor 5 x mit je 250 µl Waschpuffer gespült. Der Waschpuffer besteht aus einer Lösung aus 50 mM Tris(hydroxymethyl)aminoethan (TRIS) und 50 mM Zitronensäure, pH 7,4.

### Nachbeschichtung:

Auf den Sensor werden 50 µl inaktivierte Peroxidase (POD) gegeben. Die POD Konzentration beträgt 1 g/l. Die Lösung enthält außerdem 75 mM Na-Phosphat, 75 mM NaCl, 100 g/l Na₂SO₄. Der pH-Wert ist 6,0. Die POD Lösung wird eine Stunde bei 37 °C , bzw. über Nacht bei Raumtemperatur belassen.

### Waschschritt:

Der Überstand wird entnommen und der Sensor 5 x mit je 250 µl Waschpuffer gespült. Der Waschpuffer besteht aus einer Lösung aus 50 mM Tris(hydroxymethyl)aminoethan (TRIS) und 50 mM Zitronensäure, pH 7,4.

### Probeninkubation:

50 µl definierte Mengen an IgE enthaltendes Humanserum werden auf den Sensor gegeben und 30 min. bei 37 °C inkubiert.

### Waschschritt:

Der Überstand wird entnommen und der Sensor 5 x mit 250 µl Waschpuffer (5 mM Na-Phosphat, 85 mM NaCl, 1 g/l Tween 20, 0,5 g/l Phenol, pH 6,5) gespült.

### Regenerierung der Festphase:

250 µl einer 20 % (Gew. %) Tetrabutylammoniumhydroxidlösung werden auf den Sensor gegeben und 1 Stunde bei 37 °C inkubiert.

### Waschschritt:

Nach der Überstandsentnahme wird der Sensor 5 x mit entionisiertem Wasser gespült.

### 2. Regenerierung:

250 µl einer 1 % (Gew. %) NaBH₄-Lösung werden 15 min. bei Raumtemperatur auf dem Sensor inkubiert. Die Lösung enthält außerdem 50 mM 2-Cyclohexaminoethansulfonsäure (CHES). Der pH ist 10,0.

### Waschschritt:

Nach der Überstandsentnahme wird der Sensor 3 x mit entionisiertem Wasser und 3 x mit einer phosphatgepufferten Kochsalzlösung (pH 7,2) gespült.

### Ergebnisse (Mittelwert aus n = 2);

1. Bestimmung mit 100 lU/ml human-IgE = 829 willkürliche Einheiten (aU)
2. Bestimmung mit 100 lU/ml human-IgE = 576 aU
3. Bestimmung mit 100 lU/ml human-IgE = 623 aU
4. Bestimmung mit 0 lU/ml human-lgE = 19 aU
5. Bestimmung mit 100 lU/ml human-lgE = 767 aU
6. Bestimmung mit 100 lU/ml human-lgE (Kontrolle = unspezifischer Beschichtungsantikörper) = 14 aU

## Patentansprüche

1. Verfahren zum immunchemischen Nachweis eines Analyten in einer Probe einer biologischen Flüssigkeit, bei dem ein erster spezifischer Bindungspartner direkt an die Festphase gebunden wird, wobei die Festphase ein mit einem Edelmetall beschichteter piezoelektrischer Sensor ist, **dadurch gekennzeichnet, daß** nach erfolgter Reaktion die Festphase dadurch regeneriert wird, **daß** der erste spezifische Bindungspartner mittels eines Reagenz von der Festphase abgelöst wird.

2. Verfahren nach Anspruch 1, wobei die Festphase mit Gold beschichtet ist.

3. Verfahren nach einem der Ansprüche 1 - 2, wobei der erste spezifische Bindungspartner ein Immunglobulin oder ein Immunglobulinfragment ist.

4. Verfahren nach mindestens einem der Ansprüche 1 - 3, wobei das Reagenz ein Reduktionsmittel enthält.

5. Verfahren nach mindestens einem der Ansprüche 1 - 3, wobei das Reagenz ein Oxidationsmittel enthält.

6. Verfahren nach mindestens einem der Ansprüche 1 - 3, wobei das Reagenz NaBH₄ enthält.

7. Verfahren nach mindestens einem der Ansprüche 1 - 3, wobei das Reagenz Tetrabutylammoniumhydroxid enthält.

8. Verwendung einer regenerierbaren Festphase aus einem Edelmetall beschichteten piezoelektrischen Sensor in einem Verfahren nach Anspruch 1.

## Claims

1. Process for the immunochemical detection of an analyte in a sample of a biological fluid, in which a first specific binding partner is directly bound to the solid phase, the solid phase being a piezoelectric sensor coated with a noble metal, **characterized in that** the solid phase is regenerated, after reaction has taken place, by separating the first specific binding partner from the solid phase by means of a reagent.

2. Process according to Claim 1, wherein the solid phase is coated with gold.

3. Process according to one of Claims 1 to 2, wherein the first specific binding partner is an immunoglobulin or an immunoglobulin fragment.

4. Process according to at least one of Claims 1 to 3, wherein the reagent contains a reducing agent.

5. Process according to at least one of Claims 1 to 3, wherein the reagent contains an oxidant.

6. Process according to at least one of Claims 1 to 3, wherein the reagent contains NaBH₄.

7. Process according to at least one of Claims 1 to 3, wherein the reagent contains tetrabutylammonium hydroxide.

8. Use of a regenerable solid phase comprising a noble metal-coated piezoelectric sensor in a process according to Claim 1.

## Revendications

1. Procédé pour la détection immunochimique d'un analyte dans un échantillon d'un liquide biologique, dans lequel un premier partenaire de liaison spécifique est fixé directement sur la phase solide, la phase solide étant un capteur piézo-électrique revêtu avec un métal noble, **caractérisé en ce que**, une fois effectuée la réaction, la phase solide est régénérée par le fait que le premier partenaire de liaison spécifique est détaché de la phase solide au moyen d'un réactif.

2. Procédé selon la revendication 1, dans lequel la phase solide est revêtue avec de l'or.

3. Procédé selon la revendication 1 ou 2, dans lequel le premier partenaire de liaison spécifique est une immunoglobuline ou un fragment d'immunoglobuline.

4. Procédé selon au moins l'une des revendications 1 à 3, dans lequel le réactif contient un réducteur.

5. Procédé selon au moins l'une des revendications 1 à 3, dans lequel le réactif contient un oxydant.

6. Procédé selon au moins l'une des revendications 1 à 3, dans lequel le réactif contient NaBH₄.

7. Procédé selon au moins l'une des revendications 1 à 3, dans lequel le réactif contient de l'hydroxyde de tétrabutylammonium.

8. Utilisation d'une phase solide pouvant être régénérée, constituée d'un capteur piézo-électrique revêtu avec un métal noble, dans un procédé selon la revendication 1.
